# EUROPEAN PATENT SPECIFICATION

(11) **EP 3 773 314 B1**
(45) Date of publication and mention of the grant of the patent: **21.02.2024**
(21) Application number: 19842130.7
(22) Date of filing: 11.03.2019
(51) Int. Cl.: A61B 17/00, A61B 17/34, A61B 17/322

(54) **DIRECT AUTOMATIC TRANSFER DEVICE FOR HAIR TRANSPLANTATION**
AUTOMATISCHE DIREKTTRANSFERVORRICHTUNG ZUR HAARTRANSPLANTATION
DISPOSITIF DE TRANSFERT AUTOMATIQUE DIRECT POUR GREFFE DE CHEVEUX

(30) Priority: 09.04.2018 TR 201805012
(43) Date of publication of application: 17.02.2021
(73) Proprietor: Keçici, Lütfi, Bahçelievler/Istanbul (TR)
(72) Inventor: Keçici, Lütfi, Bahçelievler/Istanbul (TR)
(74) Representative: Tasçi, Abdurrahman
(86) International application number: PCT/TR2019/050147
(87) International publication number: WO 2020/022986

(56) References cited:
- WO-A1-2010/036808
- WO-A1-2011/091108
- AU-A1- 2013 201 087
- KR-A- 20120 019 961
- KR-B1- 101 800 365

## Description

### Technological Field:

The present invention relates to a DHI (Direct Hair Implantation) or direct hair transplantation automatic transfer device for serial transfer of pens used in hair transplantation, eyebrow transplantation, beard transplantation, and all types of graft follicle transplantation.

### Known State of the Technology:

Hair transplantation, whether due to genetic or other metabolic and structural reasons, hair loss or complete hair loss in people, hair loss is a process of permanent hair transplantation to the areas. Until today, many techniques have been applied for this purpose. In the early stages of hair transplantation, inadequate hair follicle removal, scarring, the appearance of natural discomfort, such as the distance was experienced, while today, with the developing technology, hair transplantation techniques have undergone the necessary regeneration and development. This innovative approach leads to the prevention of scars, hair follicle transplantation and natural-looking results as much as possible.

Hair transplantation process consists of various stages. These stages include the pre-application, the application itself and the post-implementation stages. Hair transplantation process in itself consists of 3 stages. These are the retrieval of the roots, the opening of the channels where the roots will be placed, and the cultivation of the roots. The hair follicles that will be sown in hair transplantation are taken from the person, especially from the scalp in the back of the head. In appropriate cases can be used in the roots taken from the back or chest area.

There are two main methods for hair root removal. These are Follicular Unit Transplantation (FUT) and Follicular Unit Extraction (FUE) methods. In the FUT technique, a portion of the scalp in the back of the patient's head is removed as a strip. The size of this strip is determined by the number of grafts needed. After planting, a small incision remains in this area. The strips obtained are divided into grafts containing 1-4 hair follicles and made ready for planting. The FUE technique is based on the acquisition of grafts with diameters of 0,9-0,5 mm with the help of an electric micro motor from the same region. Each of these grafts has 1-4 hair follicles.

There are hair transplantation pens used in hair transplantation. At the tip of the pen, there is a hair follicle to be injected into the target area. While the hair transplantation is being done, the physician injects the hair root at the tip of the pen to the target area. The pen should be immersed in sterile liquid and the hair root should be fixed again. These procedures are performed by physician assistants. The physician is forced to change the position to take the pens that are fixed to the hair root. Hence, physicians and physician assistants suffer from joint pain and adversely affect their health. At the same time, the rate of hair transplantation is adversely affected. Nowadays, the number of grafts and follicles taken in hair transplantation is small. Sclera tissue is formed during hair transplantation. The density of grafts falling to square centimeters is small. The grafts that are taken are left out for a long time due to the fact that hair transplantation is not fast and tissue death occurs. Hair transplantation can also be done with one person.

In the patent application CN1146716, "an apparatus for placing hair follicles on the conveyor belt for use in an apparatus and apparatus for conveying hair follicles"is described. A system for mechanical insertion of hair follicles into the skin has a movable charger for receiving skin grafts. The skin graft extraction apparatus has a machine that holds the skin piece in a fixed and stretched manner for skin grafts. The cooling device cools the skin part. When the skin part is held by the bench and the position where the drill rod can send the skin grafts to the charger, a hollow drill rod can be moved between positions where the drill bit can pierce skin grafts.

Patent application WO2015174645 describes the "Automatic hair seeder". The present invention relates to an automatic hair transplantation device for transporting a selected hair to the hair loss area. Transport method content: Choosing the hair where the root is healthy, without damaging the follicle; making the selected sheet having a predetermined length, placing a predetermined length of hair in a hair transplantation needle, inserting the hair transplant needle into the scalp where the hair is transferred, and then separating the hair transplantation needle from the scalp when leaving the hair.

US2015133962 describes "systems and methods for collecting, storing and transporting hair grafts". A system and method for collecting, storing and transporting biological units, in particular hair follicular units, is particularly useful for facilitating hair transplantation procedures. The system may include a placement means, a tube having a plurality of reservoirs for biological units, and one or more control mechanisms. The device and tube that store the biological units may be incorporated into the general automatic or robotic system, and may form part of a semi-automatic or manual assembly.

Patent application KR20120019961A describes the "transplantation installation of hair".

The present invention relates to transplanting apparatus is provided to prevent the fatigue of eyes and damage to a neck and a spine by enabling an operator to sit down and operate through a monitor. A vertical transfer unit is supported in a frame for vertical movement. A horizontal transfer unit is mounted on the vertical transfer unit. A finger is fixed to the horizontal transfer unit for rotation. A push unit is installed in the finger and operates a hair transplanting device by pushing the head of the hair transplanting device. A local camera is installed in the finger to magnify a scalp.

KR101800365B1 describes a device for separating hair follicles comprising a conveyor belt unit.

In the above-mentioned patent applications, methods and devices used in hair transplantation are mentioned. Although the systems described are innovations in the field of hair transplantation, there are no systems that can facilitate hair transplantation at the desired level. The products described do not allow the hair transplantation to be done quickly and cause the physician to change position continuously. In addition, these products are costly. There are occupational accidents caused by the tools when transferring the pen during hair transplantation.

As a result, a single session that can overcome the above mentioned disadvantages of 4000 to 7000 grafts can be transplanted, such as pens and so on., can reduce the number of work-related accidents due to materials, patients do not need to come back to the second session, can count the transplanted grafts, the waiting area of the grafts to the cold chain, the same patient with two physicians to provide the possibility of graft transplantation, contact with sterile water to provide continuous contact with the graft, preventing the death of the hair, a new technology is needed which minimizes material deformation as a result of the drop of hair implanter pens and low cost.

### Summary of the Invention:

The present invention is provided in claim 1, which can overcome the above-mentioned disadvantages.

In one session, 4000 to 7000 grafts can be transplanted and it can reduce the number of work-related accidents due to materials, patients do not need to come back to the second session, can count the transplanted grafts, the waiting area of the grafts to the cold chain, the same patient with two physicians to provide the possibility of graft transplantation, contact with sterile water to provide continuous contact with the graft, preventing the death of the hair, It is a new technology that minimizes material deformation as a result of the drop of hair implanter pens and low cost.

The device of the present invention provides for the transport of 4000 to 7000 grafts in a single session. The device of the present invention provides a more secure and rapid graft transplant with the design it has. The device according to the invention with pens and so on, the rate of work accidents occurring during the application of materials is reduced. With the rapid growth of hair enabled by the device of the invention, patients do not need to take a second session. Due to this situation, the cost of hotel, transfer and medicine is minimized.

The number of grafts are counted by the pen counting sensor in the device of the present invention. Thus, the number of grafts transplanted is clear. The waiting area of the grafts is suitable for the cold chain. The grafts are constantly in contact with the sterile liquid. Therefore, graft tissues are prevented from dying. The device of the present invention provides the same patient with the opportunity to plant hair by two physicians. The device of the present invention is an ergonomic increase in the work of physicians and physician assistants. In the alternative embodiment of the invention, in order for the helpers to fill the hair implanter pen to work more easily, the part in the pen collection apparatus can be divided into two and at least two physician assistants can be more comfortable to work. This process can be done by designing the conveyor belt by dividing it into two parts "V" form from the pen collection unit.

The device of the present invention provides a suitable operation by adjusting the desired angle. The transfer of hair implanter pens in the device of the present invention is safe. Therefore, the material deformation that may occur as a result of the drop of hair on a hard ground is minimized.

Because of the easy fixing of the components forming the invention, it is easy to install and the costs are low due to the short installation time. The invention also has a robust construction.

### Description of Drawings:

The invention will now be described with reference to the accompanying drawings, whereby the features of the invention will become more apparent and appreciated, but this is not intended to limit the invention to these particular embodiments. On the contrary, it is intended to encompass all alternatives and modifications which may be included in the scope of the invention as defined by the appended claims. It should be understood that the details shown are presented solely for the purpose of illustrating preferred embodiments of the present invention and for providing both the shaping of the methods and the most useful and easy-to-understand description of the rules and conceptual features of the invention. In these drawings;

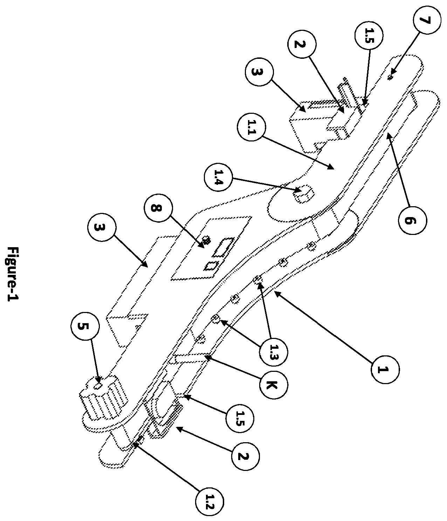

- Figure 1: is a perspective view of the system.

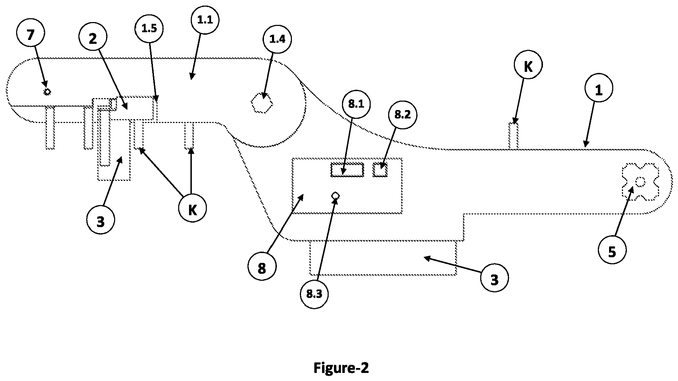

- Figure 2: is a front view of the system.

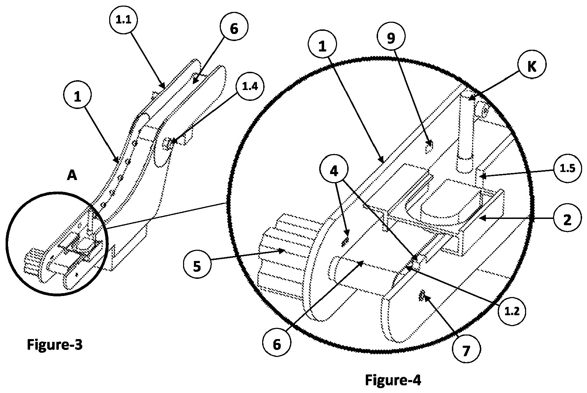

- Figure 3: is a perspective view of the system.
- Figure 4: is a view of the A view.

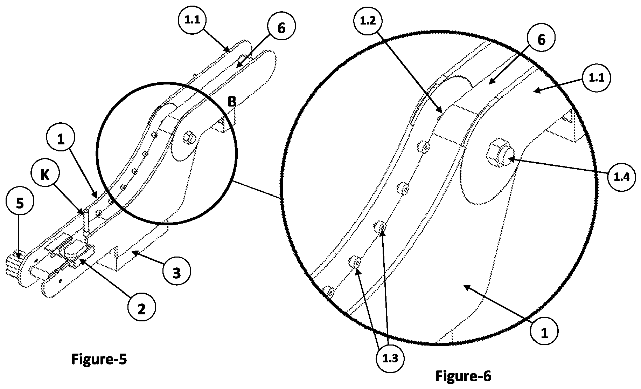

- Figure 5: is a perspective view of the system.
- Figure 6: is a view of the B view.

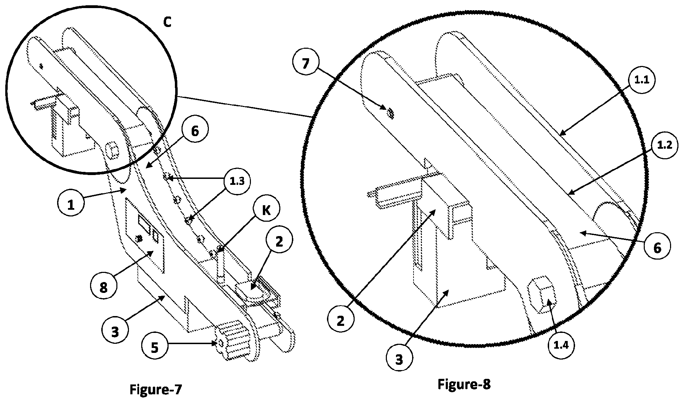

- Figure 7: is a perspective view of the system.
- Figure 8: is the view of the G view.

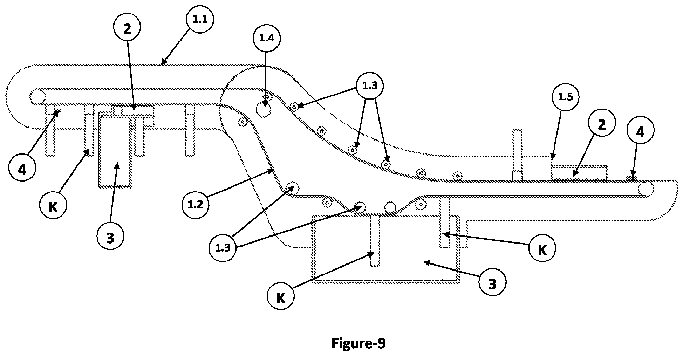

- Figure 9: is a front sectional view of the system.

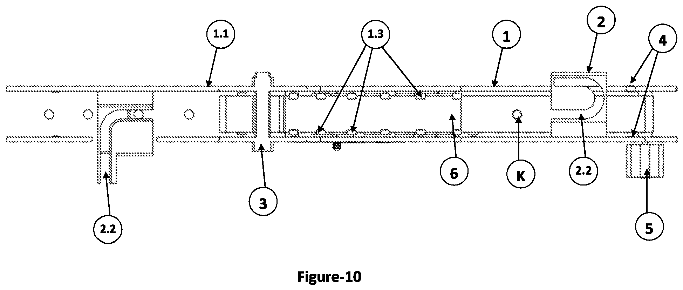

- Figure 10: is a cross-sectional view of the system.

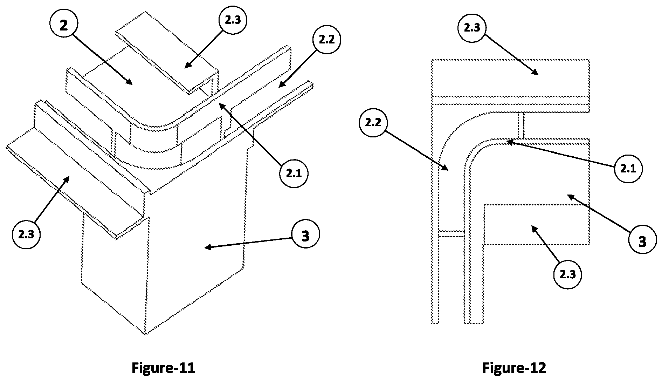

- Figure 11: is a perspective view of the pen collection apparatus.
- Figure 12: is a top view of the pen collection apparatus.

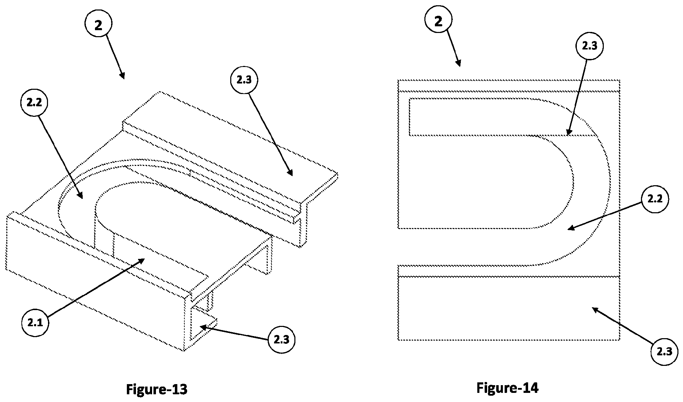

- Figure 13: is a perspective view of the pen collection apparatus.
- Figure 14: is a top view of the pen collection apparatus.

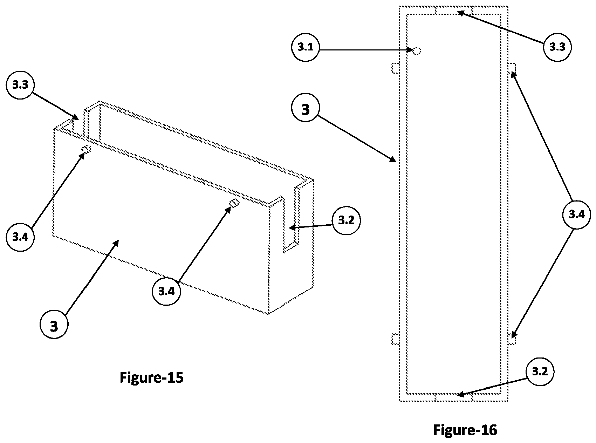

- Figure 15: is a perspective view of a sterile liquid reservoir.
- Figure 16: is a top view of the sterile liquid reservoir.

Figures to assist in the understanding of the present invention are numbered as indicated in the accompanying drawing and are given below with their names.

### Description of the References:

1. Conveyor chassis
   1. 1. Moving part
   1.2. Conveyor Belt
   1.3. Moving support pulley
   1.4. Angle adjustment element
   1.5. Fixing slot
2. Pen collection apparatus
   2.1. Magnet Part
   2.2. Collection path
   2.3. Fixing part
3. Sterile liquid reservoir
   3.1. Temperature sensor
   3.2. Input gap
   3.3. Output gap
   3.4. Fixing tab
4. Sensor
5. Engine
6. Sterile silicone coating
7. Sensor on / off button
8. Control panel
   8.1. Digital display
   8.2. Temperature display
   8.3. Speed adjustment button
9. Pen counting sensor
K. Hair implanter pen

### Description of the Invention:

The invention includes a conveyor chassis (1) having a moving part (1.1), a conveyor belt (1.2), a moving support pulley (1.3), an angle adjustment element (1.4) and a fixing slot (1.5), and a pen collection apparatus (2) positioned in the fixing slot (1.5). A is positioned on the bottom part of the pen collection apparatus (2) and on the conveyor chassis (1). A sensor (4) positioned on the conveyor chassis (1) can detect a hair implanter pen (K) located on the conveyor belt (1.2). The invention further comprises a sensor on / off button (7) that can activate the sensor (4), an engine (5) which allows the conveyor belt (1.2) to move, a sterile silicone coating (6) coated on the conveyor belt (1.2), a control panel (8) where the system is controlled from and a pen counting sensor (9) which can count the hair implanter pens (K) advancing on the conveyor belt (1.2). This sensor records the number of hair transplantations (Figure-1, Figure-3, Figure-4).

The device according to the invention has a magnet part (2.1), a collecting path (2.2) and a pen collection apparatus (2) comprising a fixing part (2.3) (Figure-11, Figure-12, Figure-13, Figure-14). In the device according to the invention, there is a sterile fluid reservoir (3) containing a temperature sensor (3.1), an input gap (3.2), an output gap (3.3) and a fixing tab (3.4) (Figure-15, Figure-16). The device of the present invention comprises a digital display (8.1), a temperature display (8.2) and a control panel (8) with a speed adjustment button (8.3) (Figure-2). The conveyor chassis (1) comprises an angle adjustment element (1.4) which ensures that the moving part (1.1) remains constant at the desired angle (Figure-1).

In the device according to the invention, the control panel (8) comprises the speed adjustment button (8.3) in which the speed of the conveyor belt (1.2) is adjusted. The pen collection apparatus (2) of the invention contains the collection path (2.2), which enables the collection of hair implanter pens (K). The pen collection apparatus (2) of the present invention has a magnet part (2.1) which enables the hair implanter pen (K) to move forward through the collection path (2.2) by applying a gripping force. The conveyor chassis (1) according to the invention contains a magnetic conveyor belt (1.2) which enables the hair implanter pen (K) to move in a fixed manner (Figure-2, Figure-3, Figure-4). The temperature sensor (3.1) of the sterile fluid reservoir (3) enables the measurement of the sterile liquid temperature. The control panel (8) of the present invention comprises the temperature display (8.2) showing the temperature information from the temperature sensor (3.1) (Figure-2, Figure-16).

### Detailed Description of the Invention:

The invention is based on; a conveyor chassis (1), a pen collection apparatus (2), a sterile fluid reservoir (3), a sensor (4), an engine (5), a sterile silicone coating (6), a sensor on / off button (7), a control panel (8) and a pen counting sensor (9) (Figure-1, Figure-3, Figure-4).

The device according to the invention has the conveyor chassis (1) comprising a moving part (1.1), a conveyor belt (1.2), a moving support pulley (1.3), an angle adjustment element (1.4) and a fixing slot (1.5). The moving part (1.1) can be brought to certain angles with the help of the angle adjustment element (1.4). The conveyor belt (1.2) contained in the device of the present invention has a magnetic property and is covered with the sterile silicone coating (6). The moving support pulleys (1.3) allow the conveyor belt (1.2) to move smoothly on a certain path. In the fixing slots (1.5) located in the device of the present invention, the pen collection apparatus (2) is fixed (Figure-3, Figure-4, Figure-5, Figure-6). The pen collection apparatus (2) consists of a magnet part (2.1), a collection path (2.2) and a fixing part (2.3). A hair implanter pen (K) is attached to the magnet part (2.1) and proceeds to the collection path (2.2). The fixing part (2.3) ensures that the pen collection apparatus (2) is fixed to the conveyor chassis (1) (Figure-10, Figure-11).

The device according to the invention has the w sterile fluid reservoir (3) which includes a temperature sensor (3.1), an input gap (3.2), an output gap (3.3) and a fixing tab (3.4). The temperature sensor (3.1) is positioned at the bottom of the sterile fluid reservoir (3).

The temperature sensor (3.1) instantly measures the temperature of the sterile liquid. In the sterile fluid reservoir (3), the hair from the input gap (3.2) enters the implanter pen (K) and exits the output gap (3.3). The sterile fluid reservoir (3) is fixed when the fixing tab (3.4) enters the fixing space on the conveyor chassis (1) (Figure-2, Figure-15, Figure-16).

The device according to the invention has the sensor (4) positioned on two head parts of the conveyor chassis (1). This sensor (4) can be opened and closed by the sensor on / off button (7). When the sensor (4) is active, the hair transplant will stop the conveyor belt (1.2) system by detecting the hair implanter pen (K) when the hair implanter pen (K) is placed on the conveyor belt (1.2) in the return path, which allows easy association with the conveyor belt (1.2). This feature is open or closed in accordance with a person who wants the hair transplantation (Figure-2, Figure-9).

In the device according to the invention, the engine (5) provides the movement of the conveyor belt (1.2). The device according to the invention has a digital display (8.1), a temperature display (8.2) and the control panel (8) with a speed adjustment button (8.3).

With digital display (8.1) the system is controlled. The temperature display (8.2) shows the temperature of the sterile fluid. The speed adjustment button (8.3) and the speed of the conveyor belt (1.2) are adjusted. The device according to the invention has the pen counting sensor (9). The pen counting sensor (9) counts the implanter pens (K) which move over the conveyor belt (1.2). The head of the hair implanter pen (K) is capable of being pulled by magnets (Figure-2, Figure-4, Figure-7, Figure-8).

The present invention is as follows; The hair is fixed to the tip of empty hair implanter pen (K). The head portion of the implanter pen (K) fixed to the hair root is left in contact with the conveyor belt (1.2) and perpendicularly. With this release, the hair implanter pen (K) is fixed to the conveyor belt (1.2). The hair implanter pen (K) fixed to the conveyor tape (1.2) proceeds with the conveyor belt (1.2). The advancing hair implanter pen (K) enters through the input gap (3.2) of the sterile fluid reservoir (3) fixed on the conveyor chassis (1).

The hair implanter pen (K) which enters the input gap (3.2) comes into the protruding part of the conveyor belt (1.2) and dips into the sterile liquid (Figure-1, Figure-2, Figure-15). The hair implanter pen (K) from the sterile liquid continues to move out of the output gap (3.3). The hair implanter pen (K), which moves with the conveyor belt (1.2), enters the collection path (2.2) on the pen collection apparatus (2). The side of the head part of the hair implanter pen (K) adhering to the collection path (2.2) adheres to the magnet part (2.1). The hair implanter pen (K) moves adhered to both the conveyor belt (1.2) and the magnet part (2.1) until the collection path (2.2). The path of collection (2.2) prevents the hair implanter pen (K) from moving with the conveyor belt (1.2) (Figure-1, Figure-2, Figure-15). With this inhibition, the magnetic bond between the conveyor belt (1.2) and the hair implanter pen (K) is broken. The magnetic bond between the hair implanter pen (K) and the magnet part (2.1) continues.

The successive hair implanter pens (K) enter the collection path (2.2) and push each other to provide progress in the collection path (2.2). The magnet part (2.1) holds the hair implanter pen (K) with the magnetic bond along the collection path (2.2). The hair implanter pen (K) contacts the sterile liquid contained in the sterile fluid reservoir (3) located below the pen collection apparatus (2) along the collection path (2.2) (Figure-1, Figure-2, Figure-15). The hair implanter pen (K) from the end of the collection path (2.2) is taken by the physician and the magnetic bond is removed by the magnet part (2.1). After applying the hair implanter pen (K) to the patient, the physician leaves the empty hair implanter pen (K) perpendicular to the magnetic conveyor belt (1.2) on the left side of the pencil collection apparatus (2). With this release, the empty hair implanter pen (K) is fixed to the conveyor tape belt (1.2). The fixed hair implanter pen (K) moves with the conveyor belt (1.2). The hair implanter pen (K) moves towards the empty pen collection apparatus (2) at the other end (Figure-1, Figure-2, Figure-15). During this progress, the pen passes and counts before the pen counting sensor (9). The hair implanter pen (K) passing through the pen counting sensor (9) enters the collection path (2.2) on the pen collection apparatus (2).

The side of the head of the hair implanter pen (K) which adheres to the collection path (2.2) adheres to the magnet part (2.1) (Figure-1, Figure-2, Figure-3, Figure-4, Figure-15). The hair implanter pen (K) moves adhered to both the conveyor belt (1.2) and the magnet part (2.1) until the collection path (2.2). The path of collection (2.2) prevents the hair implanter pen (K) from moving with the conveyor belt (1.2). With this inhibition, the magnetic bond between the conveyor belt (1.2) and the hair implanter pen (K) is broken. The magnetic bond between the hair implanter pen (K) and the magnet part (2.1) continues. The successive hair implanter pens (K) enter the collection path (2.2) and push each other to provide progress in the collection path (2.2). The magnet part (2.1) holds the hair implanter pen (K) with the magnetic bond along the collection path (2.2). The magnetic bond between the magnet part (2.1) is broken by taking the empty hair implanter pens (K), which proceed in the collection path (2.2), from the collection path (2.2). Empty hair implanter pens (K) are filled again and placed on the conveyor belt (1.2). This situation is repeated continuously during hair transplantation (Figure-1, Figure-2, Figure-15).

## Claims

1. A direct automatic transfer device for hair transplantation comprising,
- a conveyor chassis (1) with a moving part (1.1), a conveyor belt (1.2), a support pulley (1.3), an angle adjustment element (1.4) and a fixing slot (1.5);
- a pen collection apparatus (2) located in the fixing slot (1.5);
- a sterile fluid reservoir (3) located at the bottom of the pen collection apparatus (2) and on the conveyor chassis (1);
- a sensor (4) positioned on the conveyor chassis (1) configured to detect a hair implanter pen (K) located on the conveyor belt (1.2);
- a sensor on/off button (7) configured to activate the sensor (4);
- an engine (5) configured to move the conveyor belt (1.2);
- a sterile silicone coating (6) coated on the carrier belt (1.2); and
- a control panel (8) where the system is controlled and consists of a pen counting sensor (9) configured to count the hair implanter pens (K) running on the conveyor belt (1.2).

2. A direct automatic transfer device for hair transplantation as claimed in claim 1, wherein the pen collection apparatus (2) comprises a magnet part (2.1), a collection path (2.2) and a fixing part (2.3).

3. A direct automatic transfer device for hair transplantation as claimed in claim 1, wherein the sterile fluid reservoir (3) comprises a temperature sensor (3.1), an input gap (3.2), an outlet gap (3.3) and a fixing tab (3.4).

4. A direct automatic transfer device for hair transplantation as claimed in claim 1 further comprising a digital display (8.1), a temperature display (8.2), and a control panel (8) including a speed adjustment button (8.3).

5. A direct automatic transfer device for hair transplantation as claimed in claim 1, wherein the angle adjustment element (1.4) is configured to enable the moving part (1.1) to remain constant at a desired angle.

6. A direct automatic transfer device for hair transplantation as claimed in claim 4, wherein the speed adjustment button (8.3) is configured to adjust the speed of the conveyor belt (1.2).

7. A direct automatic transfer device for hair transplantation as claimed in claim 2, wherein the collection path (2.2) is configured to enable the collection of hair implanter pens (K).

8. A direct automatic transfer device for hair transplantation as claimed in claim 2, wherein the magnet part (2.1) is configured to enable the hair implanter pen (K) to travel from the collection path (2.2) by applying a gripping force to the hair implanter pen (K).

9. A direct automatic transfer device for hair transplantation as claimed in claim 1, wherein the conveyor belt (1.2) is a magnetic conveyor belt (1.2) having a magnetic feature or the conveyor belt (1.2) has a Velcro fastener configured to move the hair implanter pen (K) in a fixed manner.

10. A direct automatic transfer device for hair transplantation as claimed in claim 3, wherein the temperature sensor (3.1) is configured to measure a sterile liquid temperature.

11. A direct automatic transfer device for hair transplantation as claimed in claim 1, wherein the control panel (8) includes the temperature display (8.2) indicating the temperature information from the temperature sensor (3.1).

## Patentansprüche

1. Direkte automatische Übertragungsvorrichtung für die Haartransplantation, umfassend:
- ein Fördergestell (1) mit einem beweglichen Teil (1.1), einem Förderband (1.2), einer Stützrolle (1.3), einem Winkeleinstellungselement (1.4) und einem Befestigungsschlitz (1.5);
- eine Stiftsammelvorrichtung (2), die sich in dem Befestigungsschlitz (1.5) befindet;
- ein steriles Flüssigkeitsreservoir (3), das sich am Boden der Stiftsammelvorrichtung (2) und auf dem Fördergestell (1) befindet;
- einen Sensor (4), der auf dem Fördergestell (1) positioniert und so konfiguriert ist, dass er einen auf dem Förderband (1.2) befindlichen Haarimplantationsstift (K) erkennt;
- einen Sensor-Ein/Aus-Knopf (7), der so konfiguriert ist, dass er den Sensor (4) aktiviert;
- einen Motor (5), der so konfiguriert ist, dass er das Förderband (1.2) bewegt;
- eine sterile Silikonbeschichtung (6), die auf das Trägerband (1.2) aufgetragen ist; und
- ein Bedienfeld (8), über das das System gesteuert wird, und besteht aus einem Stiftzählsensor (9), der so konfiguriert ist, dass er die auf dem Förderband (1.2) laufenden Haarimplantationsstifte (K) zählt.

2. Direkte automatische Übertragungsvorrichtung für die Haartransplantation nach Anspruch 1, wobei die Stiftsammelvorrichtung (2) einen Magnetteil (2.1), einen Sammelweg (2.2) und einen Befestigungsteil (2.3) umfasst.

3. Direkte automatische Übertragungsvorrichtung für die Haartransplantation nach Anspruch 1, wobei das sterile Flüssigkeitsreservoir (3) einen Temperatursensor (3.1), einen Eingangsspalt (3.2), einen Ausgangsspalt (3.3) und eine Befestigungslasche (3.4) aufweist.

4. Direkte automatische Übertragungsvorrichtung für die Haartransplantation nach Anspruch 1, die ferner eine digitale Anzeige (8.1), eine Temperaturanzeige (8.2) und ein Bedienfeld (8) mit einer Taste zur Geschwindigkeitseinstellung (8.3) umfasst.

5. Direkte automatische Übertragungsvorrichtung für die Haartransplantation nach Anspruch 1, wobei das Winkeleinstellungselement (1.4) so konfiguriert ist, dass das bewegliche Teil (1.1) konstant in einem gewünschten Winkel verbleibt.

6. Direkte automatische Übertragungsvorrichtung für die Haartransplantation nach Anspruch 4, wobei der Taste zur Geschwindigkeitseinstellung (8.3) so konfiguriert ist, dass er die Geschwindigkeit des Förderbandes (1.2) einstellt.

7. Direkte automatische Übertragungsvorrichtung für die Haartransplantation nach Anspruch 2, wobei der Sammelweg (2.2) so konfiguriert ist, dass er das Sammeln von Haarimplantationsstiften (K) ermöglicht.

8. Direkte automatische Übertragungsvorrichtung für die Haartransplantation nach Anspruch 2, wobei der Magnetteil (2.1) so konfiguriert ist, dass er es dem Haarimplantationsstift (K) ermöglicht, sich aus dem Sammelweg (2.2) zu bewegen, indem er eine Greifkraft auf den Haarimplantationsstift (K) ausübt.

9. Direkte automatische Übertragungsvorrichtung für die Haartransplantation nach Anspruch 1, wobei das Förderband (1.2) ein magnetisches Förderband (1.2) mit einer magnetischen Eigenschaft ist oder das Förderband (1.2) einen Klettverschluss aufweist, der so konfiguriert ist, dass er den Haarimplantationsstift (K) in einer festen Weise bewegt.

10. Direkte automatische Übertragungsvorrichtung für die Haartransplantation nach Anspruch 3, wobei der Temperatursensor (3.1) so konfiguriert ist, dass er die Temperatur einer sterilen Flüssigkeit misst.

11. Direkte automatische Übertragungsvorrichtung für die Haartransplantation nach Anspruch 1, wobei das Bedienfeld (8) die Temperaturanzeige (8.2) enthält, die die Temperaturinformationen vom Temperatursensor (3.1) anzeigt.

## Revendications

1. Dispositif de transfert automatique direct pour la transplantation de cheveux comprenant;
- un châssis de convoyeur (1) avec une partie mobile (1.1), une bande transporteuse (1.2), une poulie de support (1.3), un élément de réglage de l'angle (1.4) et une fente de fixation (1.5);
- un appareil de collecte des stylos (2) situé dans la fente de fixation (1.5);
- un réservoir de fluide stérile (3) situé au fond de l'appareil de collecte des stylos (2) et sur le châssis du convoyeur (1);
- un capteur (4) positionné sur le châssis du convoyeur (1) configuré pour détecter un stylo implanteur de cheveux (K) situé sur la bande transporteuse (1.2);
- un bouton marche/arrêt du capteur (7) configuré pour activer le capteur (4);
- un moteur (5) configuré pour déplacer la bande transporteuse (1.2);
- une couche de silicone stérile (6) appliquée sur la bande de support (1.2); et
- un panneau de commande (8) où le système est contrôlé et qui consiste en un capteur de comptage de stylos (9) configuré pour compter les stylos implanteurs de cheveux (K) circulant sur la bande transporteuse (1.2).

2. Dispositif de transfert automatique direct pour la transplantation de cheveux selon la revendication 1, dans lequel l'appareil de collecte de stylos (2) comprend une partie aimante (2.1), un chemin de collecte (2.2) et une partie de fixation (2.3).

3. Dispositif de transfert automatique direct pour la transplantation de cheveux selon la revendication 1, dans lequel le réservoir de fluide stérile (3) comprend un capteur de température (3.1), un espace d'entrée (3.2), un espace de sortie (3.3) et une languette de fixation (3.4).

4. Dispositif de transfert automatique direct pour la transplantation de cheveux selon la revendication 1, comprenant en outre un affichage numérique (8.1), un affichage de la température (8.2) et un panneau de commande (8) comprenant un bouton de réglage de la vitesse (8.3).

5. Dispositif de transfert automatique direct pour la transplantation de cheveux selon la revendication 1, dans lequel l'élément de réglage de l'angle (1.4) est configuré pour permettre à la partie mobile (1.1) de rester constante à un angle désiré.

6. Dispositif de transfert automatique direct pour la transplantation de cheveux selon la revendication 4, dans lequel le bouton de réglage de la vitesse (8.3) est configuré pour régler la vitesse de la bande transporteuse (1.2).

7. Dispositif de transfert automatique direct pour la transplantation de cheveux selon la revendication 2, dans lequel le chemin de collecte (2.2) est configuré pour permettre la collecte des stylos implanteurs de cheveux (K).

8. Dispositif de transfert automatique direct pour la transplantation de cheveux selon la revendication 2, dans lequel la partie aimante (2.1) est configurée pour permettre au stylo implanteur de cheveux (K) de se déplacer à partir du chemin de collecte (2.2) en appliquant une force de préhension au stylo implanteur de cheveux (K).

9. Dispositif de transfert automatique direct pour la transplantation de cheveux selon la revendication 1, dans lequel la bande transporteuse (1.2) est une bande transporteuse magnétique (1.2) ayant une caractéristique magnétique ou la bande transporteuse (1.2) a une fermeture Velcro configurée pour déplacer le stylo implanteur de cheveux (K) d'une manière fixe.

10. Dispositif de transfert automatique direct pour la transplantation de cheveux selon la revendication 3, dans lequel le capteur de température (3.1) est configuré pour mesurer la température d'un liquide stérile.

11. Dispositif de transfert automatique direct pour la transplantation de cheveux selon la revendication 1, dans lequel le panneau de commande (8) comprend l'affichage de la température (8.2) indiquant les informations de température provenant du capteur de température (3.1).
